# EUROPÄISCHE PATENTSCHRIFT

(11) **EP 1 464 285 B1**
(45) Veröffentlichungstag und Bekanntmachung des Hinweises auf die Patenterteilung: **01.06.2005**
(21) Anmeldenummer: 03007470.2
(22) Anmeldetag: 04.04.2003
(51) Int. Cl.: A61B 6/00, A61B 19/00

(54) **Perspektivische Registrierung und Visualisierung interner Körperbereiche**
Perspective registration and visualisation of internal body regions
Recalage en perspective et visualisation des régions corporelles internes

(43) Veröffentlichungstag der Anmeldung: 06.10.2004
(73) Patentinhaber: BrainLAB AG, 85551 Kirchheim/Heimstetten (DE)
(72) Erfinder: Raabe, Andreas, Dr., 61352 Bad Homburg (DE); Frielinghaus, Niels, 85551 Heimstetten (DE); Witte, Jens, 80339 München (DE)
(74) Vertreter: Schwabe - Sandmair - Marx

(56) Entgegenhaltungen:
- EP-A- 1 208 808
- DE-A- 3 931 531
- DE-A- 10 015 824
- DE-A- 10 151 438
- DE-A- 19 807 884
- US-A- 5 961 456

## Beschreibung

Die Erfindung betrifft die Registrierung und Visualisierung interner Körperbereiche, Körperstrukturen und Organe im Rahmen der technischen Unterstützung medizinischer Behandlungen. Insbesondere betrifft sie die perspektivische, räumliche Darstellung von dreidimensionalen Datensätzen, Objekten oder Oberflächenmodellen in der bildgestützten Chirurgie, d. h. die Nutzbarmachung von anatomischen, dreidimensionalen Volumendatensätzen des menschlichen Körpers, die keine Oberflächenpunkte des Patientenkörpers zur Registrierung enthalten und somit bisher nicht für die Navigation verwendbar waren.

Die chirurgische Behandlung mit Hilfe bildunterstützter Navigationssysteme, auch Trackingsysteme genannt, ist grundsätzlich bekannt. Ein solches System wird beispielsweise in der DE 196 39 615 C2 und der zugeordneten US 6,351,659 B1 beschrieben. Weitere chirurgische Navigationssysteme sind beispielsweise aus der US 4,722,065, der US 4,791,934 und der US 5,383,454 bekannt. Basierend auf Daten von Bildgebungsverfahren wie Computertomographie, Kernspintomographie und Röntgendurchleuchtung nutzt eine Vielzahl von Navigationssystemen bzw. Trackingsystemen die erfassten Aufnahmen von Körperteilen zur visuellen und absoluten räumlichen Darstellung von chirurgischen Instrumenten in Relation zu Körperteilen wie Gewebe oder Knochen. Um die räumliche Darstellung von chirurgischen Instrumenten in Bezug zum Patienten innerhalb des Operationssaales zu gewährleisten, muss das Koordinatensystem der diagnostischen Daten, also beispielsweise eine Computertomographen-Schichtaufnahme, das durch die bildgebende Einheit/den Scanner festgelegt ist, mit einem zweiten Koordinatensystem abgeglichen und überlagert werden, das dem Patienten in seiner tatsächlichen Lage im Operationssaal zugeordnet ist. Die Zuordnung bzw. Ausrichtung der entsprechenden Körperstrukturen im Bilddatensatz zur tatsächlichen Patientenlage im Operationssaal wird Registrierung genannt.

Eine solche Registrierung wird gewöhnlich durch ein Überlagerungsverfahren erzielt, und zwar mit Hilfe anatomischer Landmarken oder spezieller Markierungspunkte bzw. Marker.

Mittels eines "paired point matching" oder durch ein Oberflächen-Matching von Patientenanatomie und 3D-Oberflächen des Datensatzes werden die Datensatz-Punkte den realen Punkten zugeordnet. Die im Datensatz existierenden Punkte werden mit korrespondierenden Punkten des Patienten, d. h. künstlichen oder natürlichen Oberflächenpunkten, abgeglichen. Nach erfolgtem Abgleich der Koordinatensysteme, d. h. nach einer durchgeführten Registrierung des Patienten, kann die Position eines chirurgischen Instrumentes, lokalisiert im Patientenkoordinatensystem mittels eines optischen, magnetischen oder ultraschall-basierten Lokalisierungssystems, auch im Koordinatensystem des diagnostischen Datensatzes am Computerbildschirm räumlich und positionsgenau dargestellt und zur Behandlungsunterstützung verwendet werden.

Alle bekannten Verfahren beruhen auf einer solchen Ko-Registrierung von Punkten des Bilddatensatzes (CT/MRT) und des Patienten, wobei Punkte innerhalb des Bilddatensatzes oder - raumes als absolute Bezugspunkte genutzt werden. Bei der Registrierung werden diese absoluten Bezugspunkte mit identischen Punkten des Patienten abgeglichen, die an der Patienten-"Oberfläche" erkennbar sind.

DE 101 51438 A1 offenbart ein Verfahren zur Registrierung eines größeren Bereichs durch einen Kleineren Bereich mittels Bildfusions technik.

Nachteiligerweise sind solche Registrierungsverfahren nicht in solchen Fällen anwendbar, bei denen der Bilddatensatz lediglich einen internen Körperbereich oder nur Teile der Anatomie (zum Beispiel nur Gefäße) umfasst. Das Problem liegt darin, dass solche internen Körperbereiche oftmals keine natürlichen Landmarken aufweisen (zum Beispiel im Gehirn des Patienten) und auch nicht ohne weiteres mit künstlichen Landmarken versehen werden können. Diese Volumendatensätze können deshalb mit den üblichen Methoden nicht registriert werden; sie sind aber unter anderem in der Gefäßchirurgie wichtig, wo hochauflösende Bilddatensätze ein genau definiertes, inneres Körpervolumen, in dem sich die Gefäßerkrankung (zum Beispiel Aneurysma) und die genauen Gefäßverläufe befinden, anzeigen. Bisher wurden solche Visualisierungen aus Bilddatensätzen für interne Körperbereiche lediglich separat als Informationsquelle für behandelnde Chirurgen verwendet, d. h. die aus ihnen verfügbaren Informationen wurden beispielsweise von dem Arzt vorab gesichtet, blieben jedoch ohne Nutzen, was die im Operationssaal anwesenden Patienten betraf.

Es ist die Aufgabe der vorliegenden Erfindung, ein Verfahren zur Registrierung eines Bilddatensatzes für die Visualisierung interner Körperbereiche bereitzustellen, das die oben genannten Probleme des Standes der Technik überwindet. Insbesondere soll schon grundsätzlich eine Registrierung eines Bilddatensatzes für einen internen Körperbereich zumindest insoweit ermöglicht werden, dass eine bildliche Darstellung der Informationen aus dem Bilddatensatz in für den Chirurgen nutzbarer, ausgerichteter Zuordnung zum tatsächlich im Operationssaal befindlichen Patienten möglich wird.

Diese Aufgabe wird erfindungsgemäß durch ein Verfahren gemäß dem Patentanspruch 1 gelöst. Die Unteransprüche definieren bevorzugte Ausführungsformen der vorliegenden Erfindung.

Bei dem erfindungsgemäßen Verfahren zur Registrierung eines Bilddatensatzes für die Visualisierung innerer Körperbereiche werden die folgenden Schritte durchgeführt:
- die relative Lage eines Bilderzeugungsgeräts und eines einem internen Körperbereich zugeordneten externen Körperteils wird bestimmt,
- mittels des Bilderzeugungsgerätes wird ein Bilddatensatz für den internen Körperbereich erstellt,
- die Raumlage des externen Körperteils wird bestimmt, und
- auf der Basis der relativen Lageinformationen werden die Bilddaten des internen Körperbereichs in Bezug auf die Raumlage des externen Körperteils registriert bzw. der relativen Lage nach zugeordnet.

Mit anderen Worten wird erfindungsgemäß ein externer Körperteil verwendet, um eine Brücke zu schlagen, mittels der dann die Registrierung des internen Körperbereiches ermöglicht wird. Dabei wird eine relative Positionsinformation erhalten, indem man ein relatives Positionsverhältnis zwischen externem Körperteil und Bilderzeugungsgerät bestimmt. Diese relative Information kann dann verwendet werden, um zumindest die Ausrichtung des internen Körperbereiches im realen Operationsraum zu bestimmen und den entsprechenden Bilddatensatz visuell entsprechend darzustellen. Ein solches Verfahren könnte auch "perspektivische Registrierung" bzw. insgesamt "perspektivische Navigation" genannt werden. Diese perspektivische Registrierung bzw. Navigation macht es möglich, Ausrichtungsinformationen für reale interne Körperbereiche und entsprechende Bereiche im Bilddatensatz zu erhalten, ohne dass künstliche bzw. natürliche Landmarken verwendet werden müssen.

Anwendungen stellen unter anderem gefäßchirurgische Eingriffe dar, aber auch jeder Eingriff, bei dem eine exakte Arbeitsrichtung bzw. die Richtung zum Ziel, nicht aber eine genaue Entfernung benötigt werden (zum Beispiel Aneurysmachirurgie, Angiomchirurgie, Ausrichtung von Verschraubungen, Bohrkanälen etc.). Hier ist nicht die genaue räumliche Position von Instrument und Patientenanatomie von besonderer Bedeutung, sondern die genaue augenblickliche, räumliche Orientierung in Bezug zur Blickrichtung bzw. Arbeitsrichtung des Operateurs. Somit macht die vorliegende Erfindung dreidimensionale Datensätze interner Körperbereiche für die bildgestützte Chirurgie nutzbar.

Bei einer Ausführungsform der vorliegenden Erfindung wird die relative Lage des Bilderzeugungsgeräts und des externen Körperteils in einem Koordinatensystem bestimmt, das gegenüber dem Bilderzeugungsgerät fest ist.

Gemäß der Erfindung werden zur Bestimmung der relativen Lage des Bilderzeugungsgerätes und des externen Körperteils folgende Schritte durchgeführt:
- der externe Körperteil wird mit Markierungen versehen;
- das Bilderzeugungsgerät wird aus seiner Ausgangsstellung in eine oder mehrere Positionen bewegt, in der die Markierungen in einer definierten Lage zueinander erscheinen, und
- die Bewegung des Bildaufnahmegeräts wird aufgezeichnet.

Dabei können die Markierungen derart an dem externen Körperteil angebracht werden, dass sie in einer Blickrichtung in linearer bzw. hintereinander verdeckter Anordnung erscheinen. Diese Blickrichtung ist dann reproduzierbar und eindeutig bestimmbar.

Ferner ist es möglich, die Raumlage des externen Körperteils mittels der Markierungen des externen Körperteils durch ein medizinisches Trackingsystem zu bestimmen. Die Markierungen, die verwendet werden, um die Relativlage zum Bilderzeugungsgerät zu bestimmen, können also vorteilhafterweise noch eine zweite Funktion aufweisen, die dann zum Tragen kommt, wenn eine absolute Position im Operationssaal bestimmt werden soll. Diese absolute Position ist im vorliegenden Fall die Raumlage des externen Körperteils.

Wenn, wie oben angeführt, die Bewegung des Bilderzeugungsgerätes zur relativen Lagebestimmung aufgezeichnet wird bzw. gespeichert wird, kann dies anhand von mindestens zwei Winkeln geschehen, die mindestens zwei voneinander verschiedene Stellungen definieren, in denen die Markierungen in einer definierten Lage zueinander erscheinen. Diese definierte Lage kann beispielsweise eine lineare Anordnung sein.

Gemäß einer Ausführungsform des Verfahrens nach der vorliegenden Erfindung werden die Bilddaten aus der Richtung bildlich dargestellt, aus der ein Betrachter auf den internen Körperbereich blickt. Ganz allgemein kann die Blickrichtung des Betrachters auch hier mittels eines medizinischen Trackingsystems bestimmt werden. Außerdem besteht die Möglichkeit, die Bilddaten aus der Richtung eines Instruments, insbesondere eines Mikroskops, das auf den internen Körperbereich gerichtet ist, bildlich darzustellen. Auch das Instrument kann in seiner Ausrichtung mittels eines medizinischen Trackingsystems bestimmt werden. Wenn ein chirurgisches Mikroskop verwendet wird, können vorteilhafterweise die Bilddaten in eine Bildeinspiegelungseinheit eingeblendet werden.

Besonders bevorzugt findet die Erfindung dann Anwendung, wenn der Bilddatensatz ein hochauflösender Volumenbilddatensatz eines internen Körperbereiches ist, insbesondere ein hochauflösender Röntgenbilddatensatz, vorzugsweise ein Rotationsangiographie-Bilddatensatz. Weiterhin ist die vorliegende Erfindung besonders dann von Vorteil, wenn die Bilddaten zu einer dreidimensionalen Darstellung von Strukturen, insbesondere Gefäßstrukturen, des internen Körperbereichs umgerechnet werden, die dann bildlich dargestellt werden.

Die Erfindung gestattet es, durch eine Verwendung von relativen Lageinformationen (Winkeln) und einer perspektivischen Registrierung, die Ausrichtung eines Volumendatensatzes zu ermöglichen, ohne dass dafür Punkte der Patientenoberfläche im Bilddatensatz enthalten sein müssen. Nach der Registrierung wird der Datensatz exakt in seiner Perspektive dargestellt und erlaubt die exakte Navigation einer Arbeitsrichtung und Projektion. Der Bilddatensatz wird nicht in einem absoluten Koordinatensystem erfasst, sondern in einem relativen 360°-Raum. Jede Ansicht das Datensatzes ist beispielsweise durch zwei Winkelmaße definiert, und absolute Punkte im dreidimensionalen Koordinatensystem werden nicht benötigt. Die Registrierung erfolgt unabhängig von absoluten Punkten, indem die relative Lage des erfassten Bilddatensatzes zum Patienten erfasst wird, und der Bilddatensatz wird dann bei der Registrierung mit der relativen Lage des Patienten in Übereinstimmung gebracht. Ein Chirurg kann sich mittels der Erfindung nunmehr auch anhand des visualisierten Bilddatensatzes orientieren, da dessen dargestellte Ausrichtung seiner Blickrichtung bzw. der Richtung seines Instruments entspricht, und er kann so Informationen, die ihm beim Blick auf den tatsächlichen Patienten nicht zur Verfügung stehen, aus der Visualisierung des Bilddatensatzes gewinnen. Beispielsweise kann er Gefäßverläufe vorab feststellen und diese Verläufe in seine Tätigkeit einplanen.

Die Erfindung wird im Weiteren anhand von Beispielen und einer Ausführungsform näher erläutert. In den beiliegenden Zeichnungen zeigen:
- Figur 1: eine Rotationsangiographie-Anordnung;
- Figur 2: eine Gefäßstruktur, die mittels eines dreidimensionalen Volumenbilddatensatzes dargestellt ist;
- Figur 3: die Lage der Gefäßstruktur im Kopf eines Patienten;
- Figur 4: eine Anterior-Posterior-Angiographieaufnahme mit Markerabbildungen vor der Ausrichtung;
- Figur 5: eine laterale Aufnahme mit Markern vor der Ausrichtung;
- Figur 6: einen Patientenkopf mit angebrachter Marker-Lokalisierungsvorrichtung;
- Figur 7: eine Rotationsangiographie-Anordnung in Nullstellung und in verdrehter Stellung des Scanners;
- Figur 8: eine Anterior-Posterior-Aufnahme nach der Ausrichtung;
- Figur 9: eine laterale Aufnahme nach der Ausrichtung; und
- Figuren 10 und 11: einen Scanner in Nullstellung und in verdrehter Stellung mit eingezeichneten Winkeln.

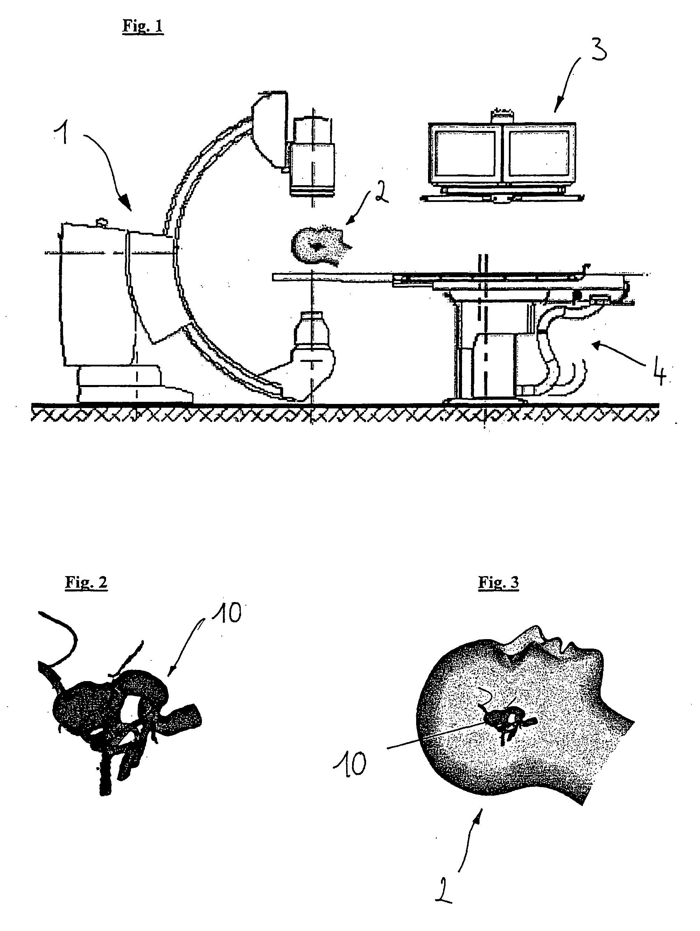

Zunächst werden die in den Figuren dargestellten Elemente näher beschrieben. In Figur 1 ist ein Bilderzeugungsgerät, nämlich ein Rotationsangiographie-Scanner 1 dargestellt, der Aufnahmen von einem Patienten machen kann, von dem nur der Kopf 2 dargestellt ist und der auf einer verstellbaren Couch 4 liegt. Im Operationssaal sind beispielsweise noch Monitore 3, zum Beispiel zur bildlichen Darstellung von anatomischen Strukturen vorgesehen. Die in Figur 2 dargestellte Gefäßstruktur 10 liegt, wie in Figur 3 zu sehen ist, im Kopf 2 des Patienten. Die Darstellung der Figur 2 entspricht einer solchen, wie sie als dreidimensionale Darstellung aus einem Volumendatensatz erhalten werden kann, wenn die Gefäße hervorgehoben und die umgebende Struktur ausgeblendet wird. Möglich wird dies meist durch die Verwendung eines Kontrastmittels für Angiographie-Aufnahmen und entsprechende Berechnungen (Computergrafik). Der Volumendatensatz mit der Gefäßstruktur 10 ist nach außen hin so begrenzt, dass er keine externen Punkte enthält, d. h. keine Punkte auf der "Oberfläche" des Patienten. Er ist ein hochauflösender Volumendatensatz, um die feinen Gefäßstrukturen sichtbar machen zu können.

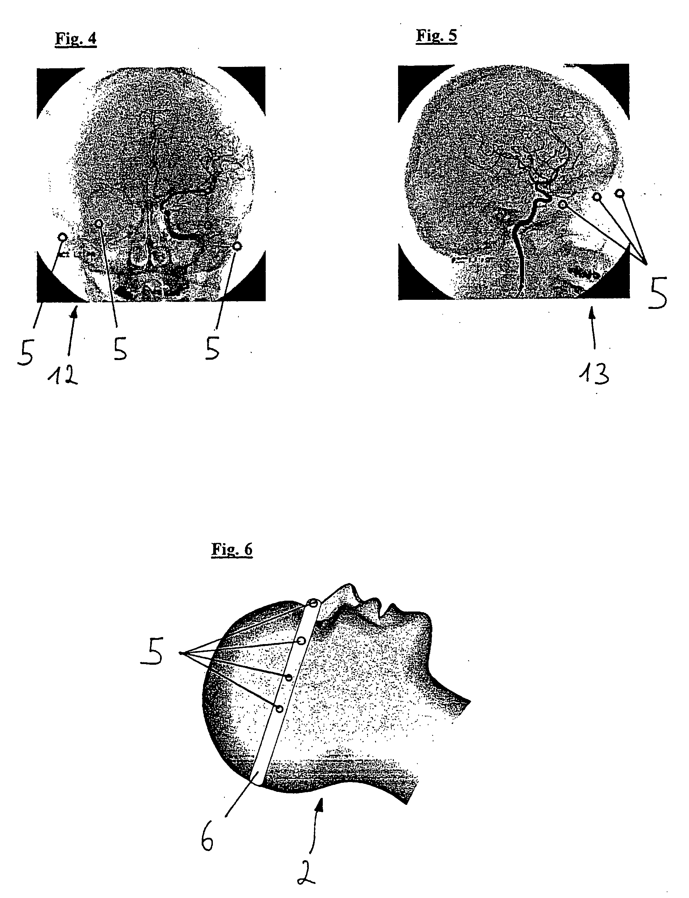

Zwei in einem rechten Winkel zueinander versetzte Angiographie-Aufnahmen, wie sie in der Nullstellung (Figur 1) des Scanners 1 gemacht werden, sind in den Figuren 4 und 5 gezeigt, wobei die Figur 4 eine Anterior-Posterior-Aufnahme und die Figur 5 eine seitliche (laterale) Links-Rechts-Aufnahme zeigt. Bei der Aufnahme war an dem Patienten eine Lokalisierungsvorrichtung 6 mit aufgesetzten Markern 5 etwa in Stirnhöhe am Kopf 2 angesetzt. In der in Figur 1 gezeigten Nullstellung des Scanners 1 zeigen die Aufnahmen 12 aus Figur 4 und 13 aus Figur 5 deshalb Markerabbildungen, die nicht in linearer Weise angeordnet sind, obwohl sie auf der Lokalisierungsvorrichtung 6 in einer Linie ausgerichtet sind.

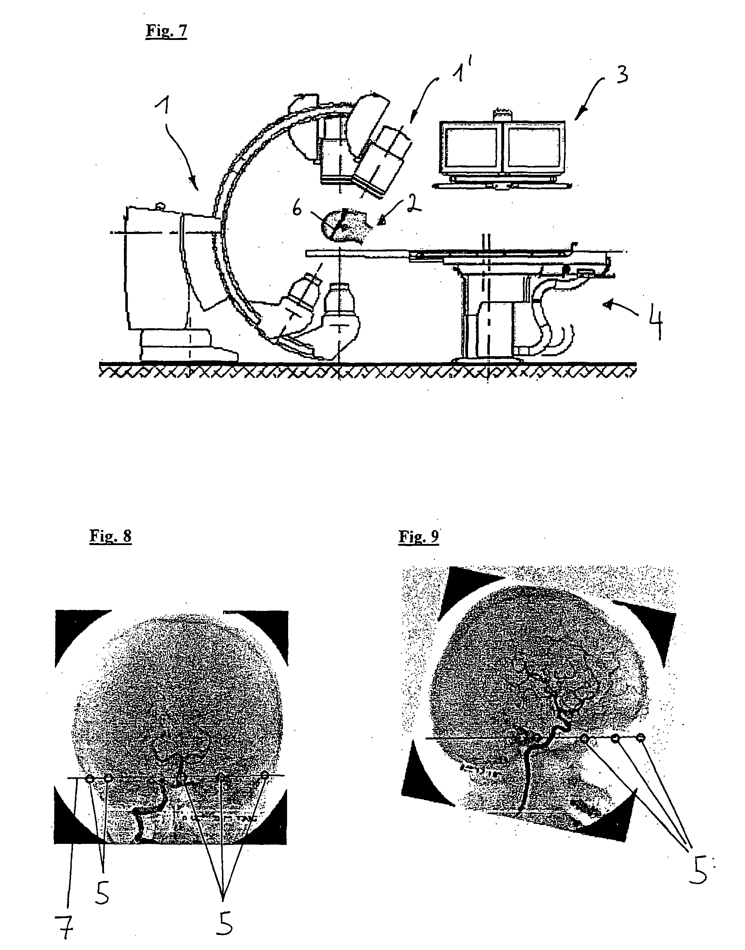

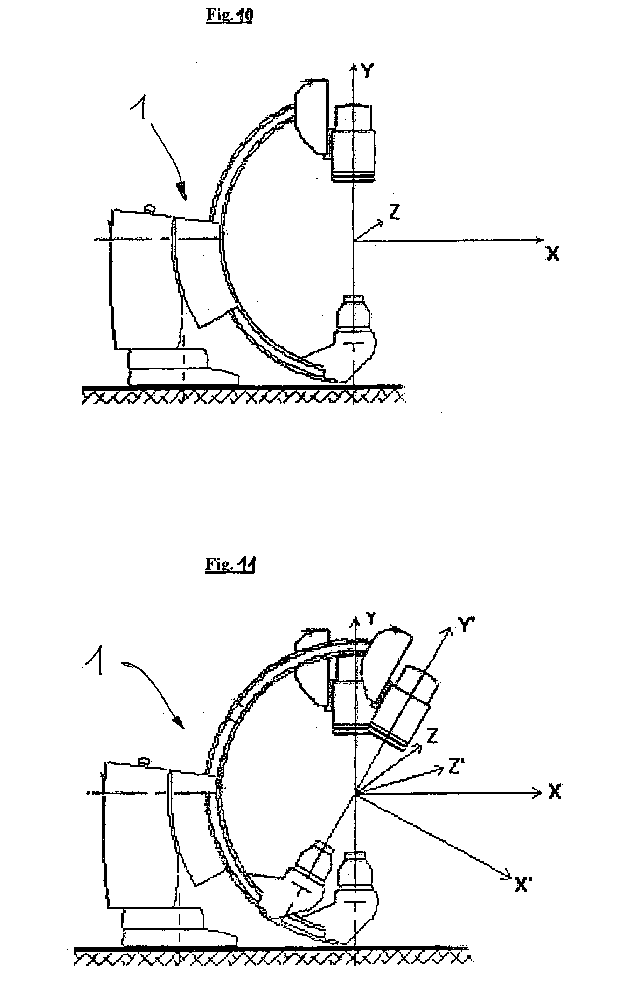

Die Figur 7 zeigt den Scanner 1 in der Rotationsangiographie-Anordnung in zwei Positionen, nämlich einmal in der Nullstellung und einmal in einer verschobenen Stellung, die mit l'bezeichnet ist. In der in Figur 7 gezeigten Stellung wurde die Aufnahme aus Figur 8 gemacht, in einer hierzu um 90° verdrehten Stellung des Scanners die Aufnahme der Figur 9, wobei in beiden Aufnahmen die Marker 5 wegen der Winkelverdrehung in linearer Ausrichtung erscheinen. Die Figuren 10 und 11 zeigen nochmals die Nullstellung (Figur 10) und eine verdrehte Stellung (Figur 11), die ganz allgemein durch eine Verdrehung aus dem Koordinatensystem X, Y, Z in ein Koordinatensystem X', Y', Z' hervorgeht.
Im Weiteren wird nun ein Ausführungsbeispiel der Erfindung detaillierter erläutert. Es ist noch ganz allgemein anzumerken, dass die in dieser Beschreibung aufgeführten Merkmale der Erfindung in jedweder Kombination umgesetzt werden können.

Gemäß der Erfindung soll zum Beispiel eine in Figur 2 dargestellte Visualisierung einer Gefäßstruktur 10 dem behandelnden Arzt immer so zur Verfügung gestellt werden, dass er sie in richtiger Ausrichtung zu seiner momentanen Blickrichtung bzw. der Richtung eines von ihm verwendeten Instruments, zum Beispiel eines Mikroskops sieht. Die durch die Struktur 10 aus dem 3D-Volumendatensatz zugänglichen Informationen kann sich der Arzt dann in direkter Weise nutzbar machen, um beispielsweise den weiteren Operationsverlauf besser planen zu können, da er in einem solchen Fall schon weiß, wie der Strukturverlauf hinter seinem momentanen Freischnitt weiterläuft. Gerade in der Gefäßchirurgie, wo Gefäßverletzungen unbedingt zu vermeiden sind, ist eine solche technische Operationshilfe von großem Vorteil.

Hierzu muss der Volumendatensatz 10 aber zunächst ausgerichtet werden, und dies ist dann möglich, wenn er registriert ist. Das Problem hierbei ist, dass der Volumendatensatz 10 keine externen Patientenlandmarken oder künstliche Landmarken aufweist, und im Folgenden wird ein erfindungsgemäßes Verfahren für diese Registrierung erläutert.

Der 3D-Volumendatensatz 10 aus der Rotationsangiographie stellt nur eine kleine, von außen zum Zweck der Registrierung nicht zugängliche Gewebestruktur dar. Deshalb wird gemäß der vorliegenden Ausführungsform der Erfindung ein vordefiniertes Winkelkoordinatensystem, zum Beispiel am Rotationsangiographie-Scanner 1, benutzt, um die räumliche Lage der Struktur 10 im dreidimensionalen Volumendatensatz zu ermitteln.

Hierzu wird zunächst die augenblickliche Lage des Patienten im Rotationsangiographie-Scanner mittels der Lokalisierungsvorrichtung 6 in Bezug zum Koordinatensystem des Scanners ermittelt. Der Scanner 1 wird, wie in Figur 7 dargestellt, so ausgerichtet, dass sich die Lokalisierungsvorrichtung in einer lateralen Probeaufnahme (Röntgenbild von rechts nach links, Figur 9) und einer Anterior-Posterior-Aufnahme (Figur 8) jeweils in genau festgelegter, vertikaler und horizontaler Ausrichtung abzeichnet. Dies ist dann der Fall, wenn die Marker 5 sich auf einer Linie befmden, die in Figur 8 beispielsweise mit dem Bezugszeichen 7 versehen ist. Die hierzu eingestellten Winkel am Rotationsangiographie-Scanner 1 werden aufgezeichnet. Diese Verstellung des Scanners 1 ist in den Figuren 10 und 11 zu sehen. Grundsätzlich kann eine solche Verdrehung auf drei Koordinatenachsen stattfinden, die von den Achsen X, Y, Z zu den Achsen X', Y', Z' führt. In bevorzugter Ausgestaltung genügt die Feststellung und Abspeicherung bzw. Aufzeichnung von zwei Winkeln, nämlich bezüglich der seitlichen Aufnahme und bezüglich der Anterior-Posterior-Aufnahme. Wichtig ist, dass die Winkeländerung festgestellt wird, sie kann in jedweder Weise aufgezeichnet, gespeichert etc. werden.

Im Anschluss hieran wird ein Rotationsangiographie-Bilddatensatz in beliebiger Winkelstellung des Scanners erstellt, und zwar ein hochauflösender Datensatz, der nur den internen Körperbereich um die Gefäßstruktur 10 herum umfasst. Dieser Bilddatensatz wird in einen die Gefäße darstellenden 3D-Volumendatensatz umgerechnet (Figur 2), wobei dieser Volumendatensatz zum Erstellungszeitpunkt am Scanner-Winkelkoordinatensystem ausgerichtet ist.

Mit Hilfe der notierten bzw. aufgezeichneten Winkel der Positionsaufnahme (Aufnahme in winkelveränderter Stellung) kann der Volumendatensatz der Rotationsangiographie nun so ausgerichtet werden, wie es der Position des Kopfes 2 (externer Körperteil) während der Rotationsangiographie entsprach. Wird im Operationssaal jetzt eine Trackingvorrichtung (nicht dargestellt) benutzt, welche anhand der Markierungen 5 die momentane Lage des Patientenkopfes 2 als absolute Lage im Raum ermitteln kann, ist somit die Lage der Patientenebene/des Patientenkoordinatensystems bekannt, und es kann nun auch ein Bezug zwischen dem Koordinatensystem des 3D-Volumendatensatzes 10 (Scanner-Koordinatensystem) und dem Patientenkoordinatensystem hergestellt werden. Die Nullstellung des Scanners kann entweder im Trackingsystem bekannt sein oder festgestellt werden. Mit anderen Worten kann jetzt durch Benutzung derselben Punkte (Lokalisierungspunkte der Lokalisierungsvorrichtung am Kopf des Patienten), die bei der Positionsaufnahme (winkelverdrehter Scanner) verwendet wurden der Volumendatensatz 10 der Rotationsangiographie mit der aktuellen Lage des Patientenkopfes 2 in Übereinstimmung gebracht werden.

Wenn nunmehr ein durch ein Trackingsystem lokalisiertes Instrument, zum Beispiel ein Operationsmikroskop (nicht dargestellt), auf den Patienten gerichtet wird, kann das 3D-Volumen mit Hilfe der Richtungsinformationen des Instrumentes räumlich und perspektivisch korrekt entsprechend der optischen Achse des Mikroskops (= Blickrichtung des Operateurs) ausgerichtet werden. Hierbei genügt es, nur die Richtungsinformation des Instrumentes festzustellen; es ist nicht nötig die genaue dreidimensionale Positionsinformation des Instrumentes zu wissen, da es für einen Arzt ausreicht, den Volumendatensatz in richtiger Ausrichtung anzusehen, um festzustellen, wie die in realiter noch verdeckten weiteren Gefäßstrukturen verlaufen. Nicht die genaue räumliche Position von Instrument und Patientenanatomie ist von besonderer Bedeutung, sondern die genaue augenblickliche, räumliche Ausrichtung des zu operierenden Gefäßes in Bezug zur Blickrichtung des Operateurs.

Wenn ein chirurgisches Mikroskop verwendet wird, kann der ausgerichtete Volumendatensatz in eine Bildeinspiegelungseinheit des Mikroskops eingeblendet werden. Er kann aber auch für den Arzt auf einer Anzeige, zum Beispiel einem Bildschirm neben dem Mikroskop zur Verfügung gestellt werden bzw. auf einem Virtual Reality Display.

## Patentansprüche

1. Verfahren zur Registrierung eines
Bilddatensatzes für die Visualisierung interner Körperbereich, bei dem der Bilddatensatz für den internen Körperbereich keine Daten über den externen Körperbereich enthält, bei dem
- die relative Lage eines Bilderzeugungsgerätes (1) und eines einem internen Körperbereich zugeordneten externen Körperteils bestimmt wird, wobei
- der externe Körperteil mit Markierungen (5) versehen wird,
- das Bilderzeugungsgerät aus seiner Ausgangsstellung in eine oder mehrere Positionen bewegt wird, in der die Markierungen (5) in einer definierten Lage zueinander erscheinen, und wobei
- die Bewegung des Bildaufnahmegeräts (1) aufgezeichnet wird,
- mittels des Bilderzeugungsgeräts (1) ein Bilddatensatz für den internen Körperbereich erstellt wird,
- die Raumlage des externen Körperteils bestimmt wird, und bei dem
- auf der Basis der relativen Lageinformation die Bilddaten des internen Körperbereichs in Bezug auf die Raumlage des externen Körperteils registriert bzw. der relativen Lage nach zugeordnet werden, wobei der Bilddatensatz aufgrund der Aufzeichnung der Bewegung des Bildaufnahmegeräts ausgerichtet wird.

2. Verfahren nach Anspruch 1, bei dem die relative Lage des Bilderzeugungsgeräts (1) und des externen Körperteils in einem Koordinatensystem (X, Y, Z) bestimmt wird, das gegenüber dem Bilderzeugungsgerät (1) fest ist.

3. Verfahren nach Anspruch 1 oder 2, bei dem die Markierungen (5) derart an dem externen Körperteil angebracht werden, dass sie in einer Blickrichtung in linearer bzw. hintereinander verdeckter Anordnung erscheinen.

4. Verfahren nach Anspruch 2 oder 3, bei dem die Raumlage des externen Körperteils mittels der Markierungen (5) des externen Körperteils durch ein medizinisches Trackingsystem bestimmt wird.

5. Verfahren nach einem der Ansprüche 2 bis 4, bei dem die Bewegung des Bilderzeugungsgeräts (1) anhand von mindestens zwei Winkeln aufgezeichnet wird, die mindestens zwei voneinander verschiedene Stellungen definieren, in denen die Markierungen (5) in einer definierten Lage zueinander erscheinen.

6. Verfahren nach einem der Ansprüche 1 bis 5, bei dem die Bilddaten aus der Richtung bildlich dargestellt werden, aus der ein Betrachter auf den internen Körperbereich blickt.

7. Verfahren nach Anspruch 6, bei dem Blickrichtung des Betrachters mittels eines medizinischen Trackingsystems bestimmt wird.

8. Verfahren nach einem der Ansprüche 1 bis 7, bei dem die Bilddaten aus der Richtung eines Instruments, insbesondere eines Mikroskops, das auf den internen Körperbereich gerichtet ist, bildlich dargestellt werden.

9. Verfahren nach Anspruch 8, bei dem die Bilddaten in eine Bildeinspiegelungseinheit eines chirurgischen Mikroskops eingeblendet werden.

10. Verfahren nach Anspruch 8 oder 9, bei dem Ausrichtung des Instruments mittels eines medizinischen Trackingsystems bestimmt wird.

11. Verfahren nach einem der Ansprüche 1 bis 10, bei dem der Bilddatensatz ein hochauflösender Volumenbilddatensatz eines internen Körperbereichs, insbesondere ein hochauflösender Röntgenbilddatensatz, vorzugsweise ein Rotationsangiographie-Bilddatensatz ist.

12. Verfahren nach einem der Ansprüche 1 bis 11, bei dem die Bilddaten zu einer dreidimensionalen Darstellung von Strukturen, insbesondere Gefäßstrukturen, des internen Körperbereiches umgerechnet werden, die dann bildlich dargestellt werden.

## Claims

1. A method for registering an image data set for visualising internal areas of the body, wherein said image data set for said internal area of the body does not contain data on the external area of the body, and wherein:
- the relative position of an imaging device (1), and of an external body part associated with an internal area of the body, is determined; wherein
- said external body part is provided with markings (5);
- said imaging device is moved from its initial position into one or more positions in which said markings (5) appear in a defined position with respect to each other; and wherein
- the movement of the imaging device (1) is recorded;
- an image data set for said internal area of the body is produced by means of said imaging device (1);
- the spatial position of said external body part is determined; and wherein
- the image data of the internal area of the body are registered, or assigned according to relative position, with respect to the spatial position of the external body part, on the basis of the relative positional information, wherein the image data set is aligned on the basis of the recording of the movement of the imaging device.

2. The method as set forth in claim 1, wherein the relative position of the imaging device (1), and of the external body part, is determined in a co-ordinate system (X, Y, Z) which is fixed with respect to the imaging device (1).

3. The method as set forth in claim 1 or 2, wherein the markings (5) are attached to the external body part in such a way that they appear in one viewing direction in a linear arrangement, or cover each other.

4. The method as set forth in claim 2 or 3, wherein the spatial position of the external body part is determined by means of the markings (5) of the external body part, using a medical tracking system.

5. The method as set forth in any one of claims 2 to 4, wherein the movement of the imaging device (1) is recorded on the basis of at least two angles which define at least two positions which are different from each other, in which the markings (5) appear in a defined position with respect to each other.

6. The method as set forth in any one of claims 1 to 5, wherein the image data are represented visually from the direction from which an observer looks onto the internal area of the body.

7. The method as set forth in claim 6, wherein the viewing direction of the observer is determined by means of a medical tracking system.

8. The method as set forth in any one of claims 1 to 7, wherein the image data are represented visually from the direction of an instrument, in particular a microscope, pointed at the internal area of the body.

9. The method as set forth in claim 8, wherein the image data are superimposed in an image inter-reflecting unit of a surgical microscope.

10. The method as set forth in claim 8 or 9, wherein the alignment of the instrument is determined by means of a medical tracking system.

11. The method as set forth in any one of claims 1 to 10, wherein the image data set is a high-resolution volume image data set of an internal area of the body, in particular a high-resolution x-ray image data set, preferably a rotational angiography image data set.

12. The method as set forth in any one of claims 1 to 11, wherein the image data are converted to a three-dimensional representation of structures, in particular vascular structures, of the internal area of the body, which are then represented visually.

## Revendications

1. Procédé de recalage d'un jeu de données d'image pour la visualisation de régions corporelles internes, pour lequel le jeu de données d'image pour la région corporelle interne ne contient pas de données sur la région corporelle externe, pour lequel
- on détermine la position relative d'un appareil d'imagerie (1) et d'une partie du corps externe correspondant à une région corporelle interne,
- la partie du corps externe étant pourvue de marquages (5),
- l'appareil d'imagerie étant amené de sa position de départ dans une ou plusieurs positions, dans laquelle les marquages (5) apparaissent dans une position définie l'un par rapport à l'autre, et
- le mouvement de l'appareil de prise de vues (1) étant enregistré,
- un jeu de données d'image est réalisé pour la région corporelle interne à l'aide de l'appareil d'imagerie (1),
- la position spatiale de la partie du corps externe est déterminée et pour lequel,
- sur base des informations de position relative, les données d'image de la région corporelle interne sont recalées par rapport à la position spatiale de la partie du corps externe ou affectées à la position relative, le jeu de données d'image étant orienté sur base de l'enregistrement du mouvement de l'appareil de prise de vues.

2. Procédé suivant la revendication 1, pour lequel on détermine la position relative de l'appareil d'imagerie (1) et de la partie du corps externe dans un système de coordonnées (X, Y, Z) qui est fixe par rapport à l'appareil d'imagerie (1).

3. Procédé suivant la revendication 1 ou 2, pour lequel les marquages (5) peuvent être appliqués de telle manière à la partie du corps externe qu'ils apparaissent selon une disposition linéaire ou cachés l'un derrière l'autre dans une direction d'observation.

4. Procédé suivant la revendication 2 ou 3, pour lequel on détermine la position spatiale de la partie du corps externe à l'aide des marquages (5) de la partie du corps externe à l'aide d'un système de suivi pour applications médicales.

5. Procédé suivant l'une des revendications 2 à 4, pour lequel le mouvement de l'appareil d'imagerie (1) est enregistré à l'aide d'au moins deux angles qui définissent au moins deux positions différentes l'une de l'autre, dans lesquelles les marquages (5) apparaissent dans une position définie l'un par rapport à l'autre.

6. Procédé suivant l'une des revendications 1 à 5, pour lequel les données d'image sont représentées en image depuis la direction dans laquelle un observateur regarde la région corporelle interne.

7. Procédé suivant la revendication 6, pour lequel on détermine la direction d'observation de l'observateur à l'aide d'un système de suivi pour applications médicales.

8. Procédé suivant l'une des revendications 1 à 7, pour lequel les données d'image sont représentées en image depuis la direction d'un instrument, en particulier d'un microscope, qui est orienté sur la région corporelle interne.

9. Procédé suivant la revendication 8, pour lequel les données d'image sont affichées dans une unité de projection d'image d'un microscope chirurgical.

10. Procédé suivant la revendication 8 ou 9, pour lequel on détermine l'orientation d'un instrument à l'aide d'un système de suivi pour applications médicales.

11. Procédé suivant l'une des revendications 1 à 10, pour lequel le jeu de données d'image est un jeu de données d'images spatiales à haute résolution d'une région corporelle interne, en particulier un jeu de données d'image radiographique à haute résolution, de préférence un jeu de données d'image d'angiographie dynamique en rotation.

12. Procédé suivant l'une des revendications 1 à 11, pour lequel les données d'image sont converties en une représentation tridimensionnelle de structures, en particulier de structures vasculaires, de la région corporelle interne, qui sont alors représentées en images.
